# EUROPEAN PATENT APPLICATION

(11) **EP 4 759 240 A1**
(43) Date of publication of application: **17.06.2026**
(21) Application number: 24219670.7
(22) Date of filing: 13.12.2024
(51) Int. Cl.: A61B 6/10, A61B 6/51, A61B 6/46, A61B 6/00

(54) **METHOD FOR X-RAY IMAGING OF A SMALL VOLUME**

(71) Applicant: Dentsply Sirona Inc., York, PA 17401 (US); SIRONA Dental Systems GmbH, 64625 Bensheim (DE)
(72) Inventor: Elvers, Michael, 64625 Bensheim (DE); Ulrici, Johannes, 64625 Bensheim (DE); Hieber, Simone, 64625 Bensheim (DE); Voss, Björn, 64625 Bensheim (DE); Kim, Hong Keun, Bensheim (DE); Klein, Konrad, Bensheim (DE)
(74) Representative: Aldridge, Henry Alexander

(57) **Abstract**

The present invention relates to method of generating an x-ray image using an x-ray system (1) comprising an x-ray device (2) which includes: an x-ray detector (4; 14); an x-ray source (3) for emitting a x-ray cone beam; an x-ray collimator (12; 15) having one or more adjustable shutters (25) for setting an aperture (24) for the x-ray radiation; and a first reference point (P1) for positioning a patient's oral cavity. The method comprises the step of: acquiring a digital impression (DI) of the patient's oral cavity; selecting a region of interest (ROI) in the digital impression (DI); determining the size of the region of interest; determining a second reference point (P2) in the digital impression; determining the position of the selected region of interest relative to the x-ray device (2) using the first reference point, the second reference point, and the digital impression; setting the positions of the x-ray detector and the x-ray source on opposing sides of the patient's head so that the region of interest can be exposed with x-ray cone beam radiation and imaged with the x-ray detector; adjusting the shutters (25) so that a size of the aperture (24) restricts the x-ray cone beam to expose substantially not more than the region of interest (ROI) for the set positions of the x-ray detector and the x-ray source; and acquiring at least one x-ray projection image of the region of interest (ROI) at the set positions of the x-ray detector and the x-ray source with the correspondingly adjusted shutters (25); generating an x-ray image by using the x-ray projection images.

## Description

### TECHNICAL FIELD OF THE INVENTION

The present invention relates to a system and a method for x-ray imaging in the dental field.

### BACKGROUND OF THE INVENTION

A cephalometric (CEPH) image (cephalogram), an orthopantomogram (OPG) which is generally known as panoramic image, and digital volume tomographic (DVT) image which is generally known as a 3D image are part of an orthodontist's standard diagnostics. These images are required, among other things, to determine relevant positions in the patient's jaw for the planning, implementation, and follow-up of an orthodontic treatment.

For example, selecting a small volume for a 3D image is particularly important in endodontics. The 3D images provide the practitioner with important information about the position and course of the root canals which can be crucial for the success of the treatment.

The difficulty with an image limited to a single tooth lies, on the one hand, in the exact positioning of the volume and, on the other hand, in the precise execution of the x-ray imaging with the small volume.

### DISCLOSURE OF THE INVENTION

An objective of the present invention is to provide a method and system for x-ray imaging of a small volume, namely a region of interest in the oral cavity of the patient.

These objectives are achieved by the method as defined in claim 1, and the x-ray system as defined in claim 8. The subject-matters of the dependent claims define further developments and preferred embodiments.

The method according to the invention is a computer-implemented method and is used to generate an x-ray image using an x-ray system comprising an x-ray device which includes: an x-ray detector; an x-ray source for emitting an x-ray cone beam; and an x-ray collimator having one or more adjustable shutters for setting an aperture for the x-ray radiation. Herein the generated x-ray image is not limited to a specific kind, and can be a 3D volume image, a tomosynthesis image, or a cephalometric image. The method comprises the following steps: providing a first reference point for positioning a patient's oral cavity, acquiring a digital impression of the patient's oral cavity; selecting a region of interest (ROI) (namely a small volume) in the digital impression; determining the size of the region of interest; determining a second reference point in the digital impression; determining the position of the selected region of interest relative to the x-ray device using the first reference point, the second reference point, and the digital impression; setting the positions of the x-ray detector and the x-ray source on opposing sides of the patient's head so that the region of interest can be exposed with x-ray cone beam radiation and imaged with the x-ray detector; adjusting the shutters so that a size of the aperture restricts the x-ray cone beam to expose substantially not more than the region of interest for the set positions of the x-ray detector and the x-ray source; and acquiring at least one x-ray projection image of the region of interest at the set positions of the x-ray detector and the x-ray source with the correspondingly adjusted shutters; generating an x-ray image by using the at least one x-ray projection image.

In a preferred embodiment, the tube voltage of the x-ray source and/or the thickness of the shutters are configured such that the shutters don't entirely absorb the x-ray radiation during the acquisition of the one or more x-ray projection images. Since the x-ray detector has a large area, the ROI will be imaged more intensely than the surrounding dental structures. Thereby the dentist can also view the surrounding dental structures of the ROI while preventing the patient from unnecessary x-ray dose. Thereby the x-ray projection images include a first area showing the selected region of interest, and a second area showing an area surrounding the selected region of interest.

In another preferred embodiment, the tube voltage of the x-ray source and/or the thickness of the shutters are configured such that the shutters entirely absorb the x-ray radiation during the acquisition of the one or more x-ray projection images. Thereby the x-ray projection images show only the selected region of interest. And the patient can be prevented from receiving an unnecessary x-ray dose in as much as possible.

In another preferred embodiment, during the acquisition the x-ray detector and the x-ray source are moved along a curvilinear trajectory or a circular trajectory. And a plurality of x-ray projection image of the region of interest are acquired respectively at a plurality of set positions, along the said trajectory, of the x-ray detector and the x-ray source with the correspondingly adjusted shutters. By using the plurality of x-ray projection image, it is possible to reconstruct a 3D volume image, or alternatively a panoramic image.

In another preferred embodiment, during the acquisition the x-ray detector and/or the x-ray source are moved along a linear trajectory. And a plurality of x-ray projection images of the region of interest are acquired respectively at a plurality of set positions, along the said trajectory, of the x-ray detector and the x-ray source with the correspondingly adjusted shutters. By using the plurality of x-ray projection images, it is possible to reconstruct a cephalometric image.

The present invention also includes a computer program having computer-readable codes which, when executed by a computerized x-ray system, causes the said system to perform the method. The computer program can be stored on a storage medium as imaging software.

The present invention also includes an extraoral x-ray system having an x-ray source; an x-ray detector; and a collimator; and a control means for controlling the x-ray source, the x-ray detector, and the x-ray collimator. And the control means is configured to perform the method.

An advantageous effect of the invention is that the method is suitable for reducing the risk of misdiagnosis and incorrect treatment. In sum, the method according to the invention creates diagnostic and therapeutic added value for the dentist through more reliable diagnosis.

### BRIEF DESCRIPTION OF THE DRAWINGS

In the following description, the present invention will be explained in more detail with reference to the exemplary embodiments and with reference to the drawings, wherein.
- Fig. 1 -: shows a schematic drawing of an extraoral x-ray system with a CEPH unit according to an embodiment of the present invention;
- Fig. 2 -: shows a schematic drawing of an extraoral x-ray system without a CEPH unit according to an embodiment of the present invention;
- Fig. 3 -: shows a schematic drawing of a computerized imaging/manufacturing system with an intra oral scanner, an MRI device, and subtractive/additive manufacturing devices according to an embodiment of the present invention;
- Fig. 4 -: shows a schematic drawing of a milling machine according to an embodiment of the present invention;
- Fig. 5 -: shows a schematic drawing of a collimator according to an embodiment of the present invention;
- Fig. 6 -: shows another schematic drawing of the collimator according to an embodiment of the present invention;
- Fig. 7 -: shows a schematic drawing of an intra oral scanner according to an embodiment of the present invention, while the dentist acquiring a digital impression of the oral cavity of a patient;
- Fig. 8 -: shows a schematic drawing of the detector when exposed during imaging according to an embodiment of the present invention, wherein the dashed lines shows the contour corresponding to the adjusted shutters;
- Fig. 9 -: shows a schematic drawing of a digital impression according to an embodiment of the present invention;

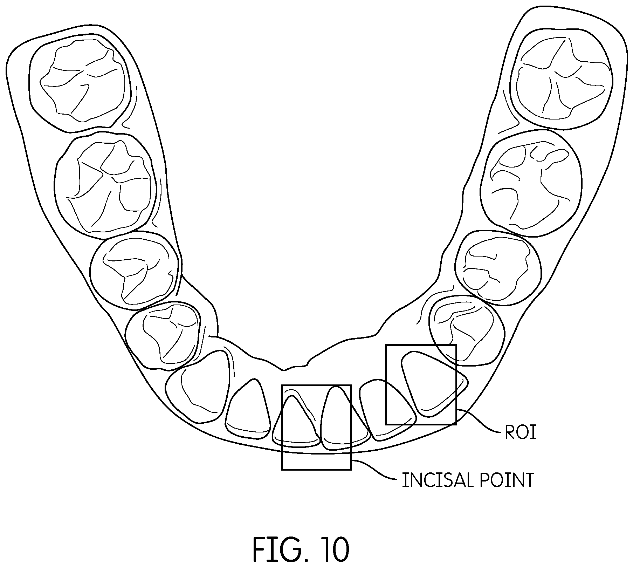

- Fig. 10 -: shows another schematic drawing of the digital impression according to an embodiment of the present invention;

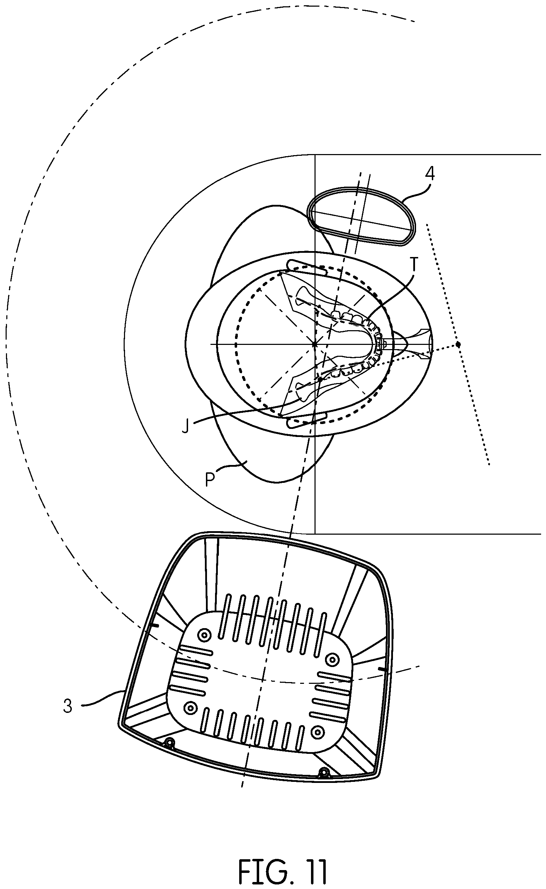

- Fig. 11 -: shows a schematic drawing of the trajectories of the x-ray detector and the x-ray source during imaging.

The reference numbers shown in the drawings designate the elements listed below, which are referred to in the following description of the exemplary embodiments.
1. Extraoral X-ray system
   1' Imaging System
2. X-ray device
3. X-ray source
4. Primary x-ray detector (PAN/DVT detector)
5. Operating unit (user interface)
6. 6.' Head fixation
7. Bite block
8. Computing unit (Computer)
9. Display device (Screen)
10. Input device (Keyboard)
11. Input device (Mouse)
12. Primary collimator (dashed lines)
13. Cantilever arm
14. Secondary x-ray detector (CEPH detector)
15. Secondary collimator (CEPH collimator)
16. MRI Imaging device
17. 3D Printing unit
18. Post Processing unit
19. Milling machine
20. Dental Block
21. Dental tool
22. Carriage
23. Display
24. Aperture
25. Collimator Blades/shutter
26. Intraoral optical Scanner

DI: Digital Impression
ROI: Region of Interest in DI
P1: First reference point
P2: Second reference point
J: Jawbone of the patient

### DETAILED DESCRIPTION OF THE EMBODIMENTS

In the following description, various imaging systems (1,1') and methods according to the present invention will be explained in more detail.

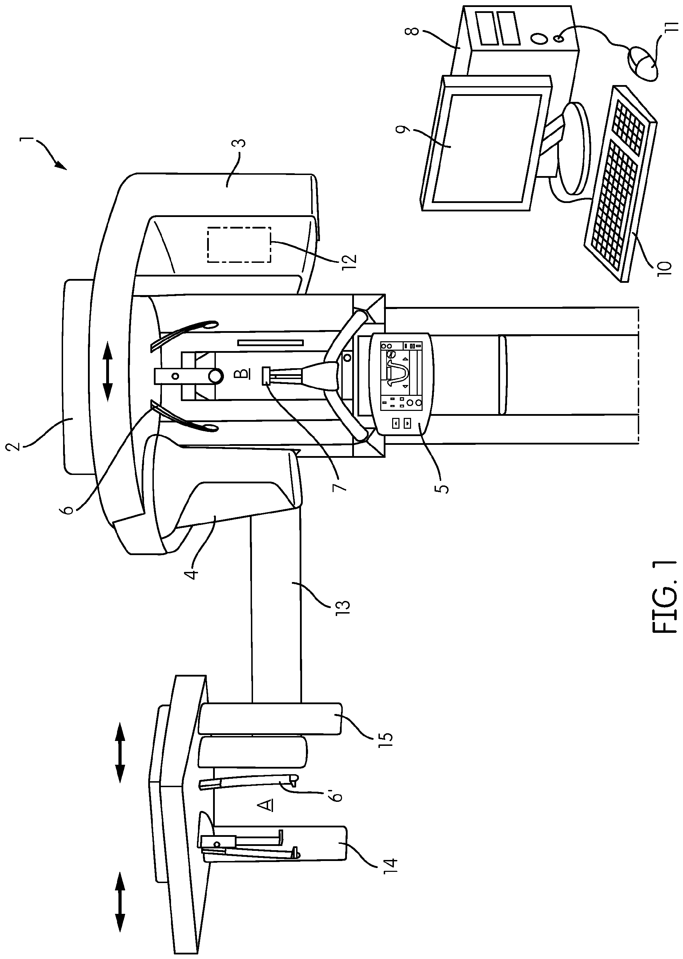

The methods according to the present invention, which are explained in more detail below, are computer-implemented methods (i.e., herein also referred to as software) and can be carried out on computer assisted systems (1,1') as shown in the embodiments in Figs. 1-3.

The present invention also includes corresponding computer programs (i.e., the software) having computer-readable code for implementing the methods. The computer program is provided on a computer readable storage medium accessible to the systems (1,1').

The computerized extraoral x-ray system (1) shown in Fig. 1 is a multifunctional system for panoramic imaging, digital volume tomographic imaging, and cephalometric imaging.

The extraoral x-ray system (1) comprises an x-ray source (3) and a primary x-ray detector (4) that are rotatably arranged around the patient's head for PAN/DVT imaging (see position B in Fig. 1). The x-ray source (3) and the x-ray detector (4) are on a gantry. The x-ray source (3) has a primary collimator (12). The extraoral x-ray system (1) also has a cantilever arm (13) that holds a secondary x-ray detector (14) (i.e., the ceph detector) and a secondary collimator (15) (i.e., the ceph collimator). Preferably a CMOS-based ceph detector is used. This CMOS-based ceph detector is preferably a detector with a scintillator or a direct converting detector. The x-ray ceph detector (14) and the x-ray ceph collimator (15) can be linearly moved along the patient's head (see position A in Fig. 1). A sequence of projection images of the patient's head can be obtained by exposing the patient's head with x-rays from the x-ray source (3) while linearly moving the x-ray ceph detector (14) and the x-ray ceph collimator (15) on opposite sides along the patient's head, while the head being positioned between them. See Position A in Fig. 1 for the patient's head in the ceph mode. In the ceph mode, the primary x-ray detector (4) is automatically moved out of the x-ray beam path to a side position to avoid blocking the x-rays that are emitted from the x-ray source (3) towards the ceph collimator (15). The extraoral x-ray system (1) has a control means for energizing the x-ray source (3), and linearly moving the x-ray ceph detector (15), and the x-ray ceph collimator (15). The patient's head can be positioned in the x-ray ceph system (1) with a head fixation (6'). The x-ray ceph collimator (15) has a ceph aperture structure. The x-ray ceph collimator (15) is provided as an adjustable (motorized) multicomponent aperture structure, wherein the vertically extending aperture blade components can be moved sideways by a motorized mechanism to adjust the width of the aperture, wherein the horizontally extending aperture blade components can be moved up/down by a motorized mechanism to adjust the height of the aperture. Said components can be made from x-ray opaque/absorbing material.

In the PAN/DVT mode, the trajectory of the x-ray source (3) and the x-ray detector (4) during the PAN/DVT imaging can describe a circular path. However, it can also assume a form deviating from this. If several actuators (not shown) are controlled simultaneously, a trajectory deviating from a pure circular path around the patient's head can be achieved. The various possible trajectories (*course*) of the x-ray source (3) and the x-ray detector (4) with respect to the bite block (7) and the head fixation (6) are calibrated for the system (1). The patient's head can be positioned with a bite block (7) and optionally with a head fixation (6). See Position B in Fig. 1 for the patient's head in the PAN/DVT mode. The trajectory (*course*) of the x-ray source (3) and the primary x-ray detector (4) with respect to the bite block (7) and the head fixation (6) are known by the system (1). The primary x-ray detector (4) detects the x-rays emitted by the x-ray source (3) during the rotation. The x-ray projection images for PAN/DVT imaging are acquired, namely read out from the x-ray detector (4). The Primary x-ray detector (PAN/DVT detector) has a separate PAN detector and a separate DVT detector which can be rotated around the vertical axis to face the x-ray source (3) in accordance with the PAN/DVT imaging respectively. Or it could be a single flat panel detector capable for PAN as well as DVT.

Flat panel sensor technologies of x-ray detectors (4) for dental applications are based either on TFT (e.g. amorphous silicon or IGZO) or, depending on the size, on one or more CMOS wafers. Cesium iodide (CsI) is usually used as the scintillator material. They can be used in 1x1 binning for panorama imaging or e.g., 2x2 binning for DVT. The readout region of the flat-panel detectors can be predefined (so-called partial mode). The larger the area to be read out, the lower the resulting frame rate. The dynamic range can be 16 bits. Multiple gain factors can be set and selected depending on the application.

The computerized extraoral x-ray system (1) also comprises: an operating unit (5) such as a user interface for controlling all functionalities of the imaging modes; a computing unit (8) e.g., a computer; and a display device (9) such as a screen for visualizing any data sets (projections images and the like) resulting from the software. The CEPH/PAN/DVT modes can be each selected by the user e.g., through the operating unit (5) and/or the computer unit (8). The computer may be connected to the extraoral x-ray system (1) via a local area network (not shown) or, alternatively, via the Internet. The computer is connected to input devices such as a keyboard (10), mouse (11), and the like. The computer may be also part of a cloud. Alternatively, the computer may be integrated into the extraoral x-ray system (1). Alternatively, all or some of the computations may take place in the cloud as cloud computing or on an FPGA (field programmable gate array) in hardware. The computer executes the computer program and provides the data sets, e.g., the cephalometric images, the panoramic images, and/or the DVT images for visualization on the screen. The screen may be provided spatially separate from or integrated with the extraoral x-ray system (1). Preferably, the computer may also control all functions of the extraoral x-ray system (1). Alternatively, separate computers may be used for the control, operation, and image reconstruction.

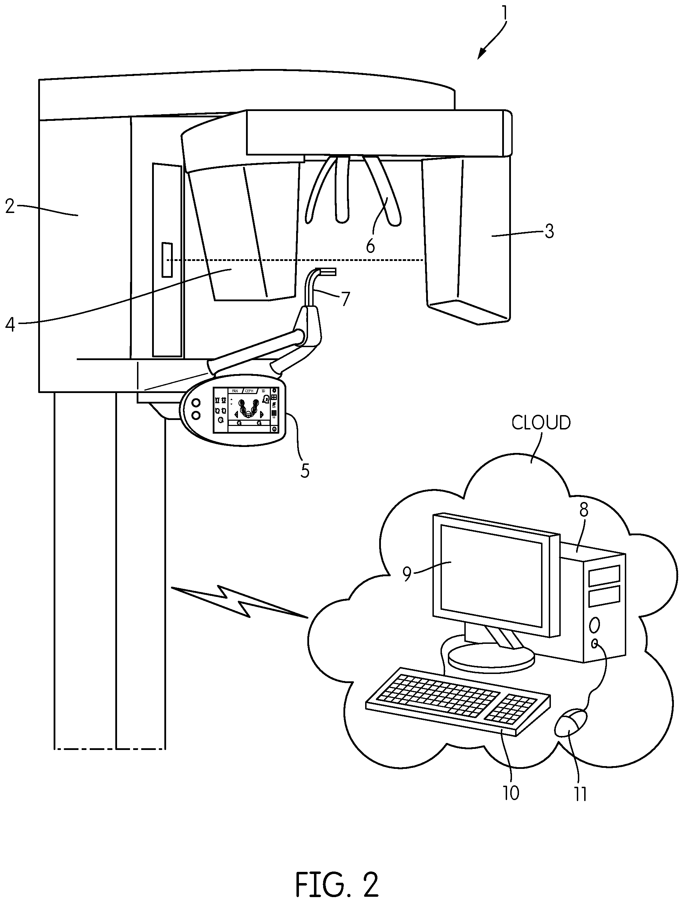

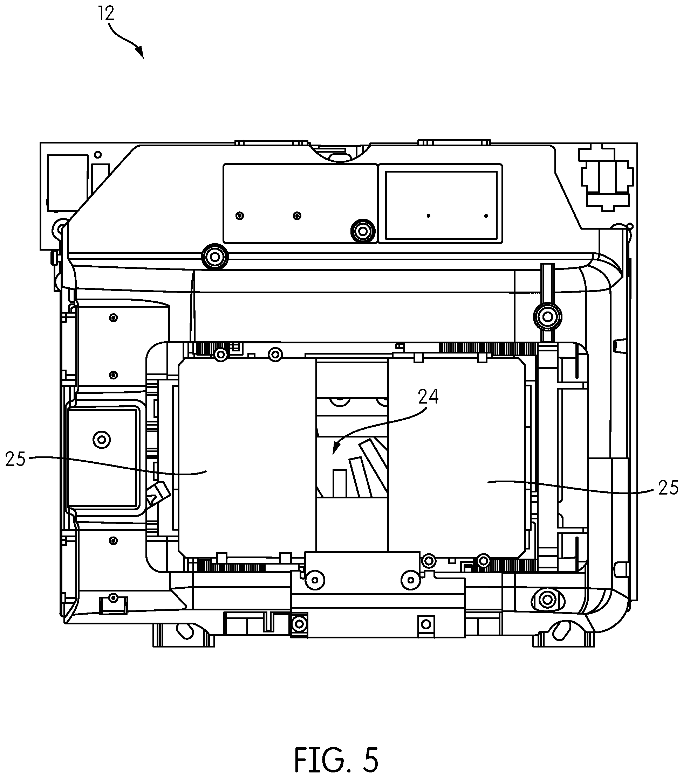

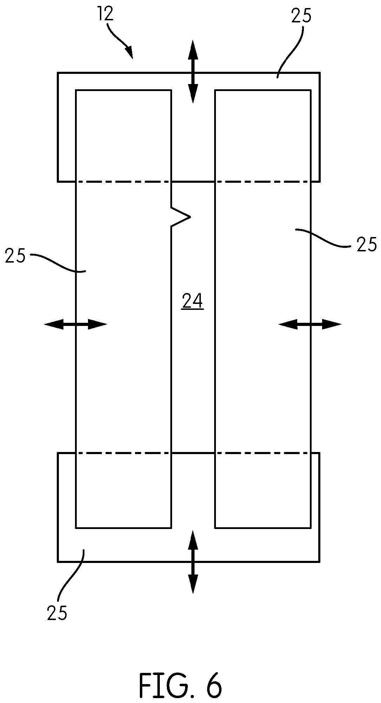

The computerized extraoral x-ray system (1) shown in Fig. 2 is a multifunctional system for panoramic imaging, digital volume tomographic imaging, and virtual cephalometric imaging. The computerized extraoral x-ray system (1) shown in Fig. 2 differs from the x-ray system (1) Fig. 1 only in that it has no ceph unit attached on a cantilever arm (13). The virtual cephalometric imaging can be performed by back projection with parallel beam geometry using a 3D Volumes image reconstructed from 2D x-ray projection images acquired during revolution. The computerized extraoral x-ray system (1) shown in Fig. 2 has a primary collimator (dashed lines) (12) which is shown in more detail in Fig. 5 and Fig. 6. The x-ray collimator (12) is positioned between the patient's head and the x-ray source (3). The x-ray collimator (12) is attached into the housing of the x-ray source (3) so that they move/rotate together. The collimator (12) has an x-ray aperture (24) to restrict the x-ray cone beam angle emitted from the x-ray source (3). The width/height of the x-ray aperture (24) is adjustable for varying the x-ray cone beam angle. The x-ray collimator (12) is provided as an adjustable (motorized) multicomponent aperture structure as shown in Fig. 6, wherein the vertically extending aperture blade (25) components can be moved sideways by a motorized mechanism to adjust the width of the aperture (24), wherein the horizontally extending aperture blade (25) components can be moved up/down by a motorized mechanism to adjust the height of the aperture (24). Said components can be made from x-ray opaque/absorbing material. The x-ray ceph collimator (15) and the x-ray collimator (12) are similar in structure and functionality. The 2D x-ray projection images are acquired, namely read out from the readout regions of x-ray flat panel detector (4) with a predetermined frame rate. This can be set and variably controlled by the x-ray system (1).

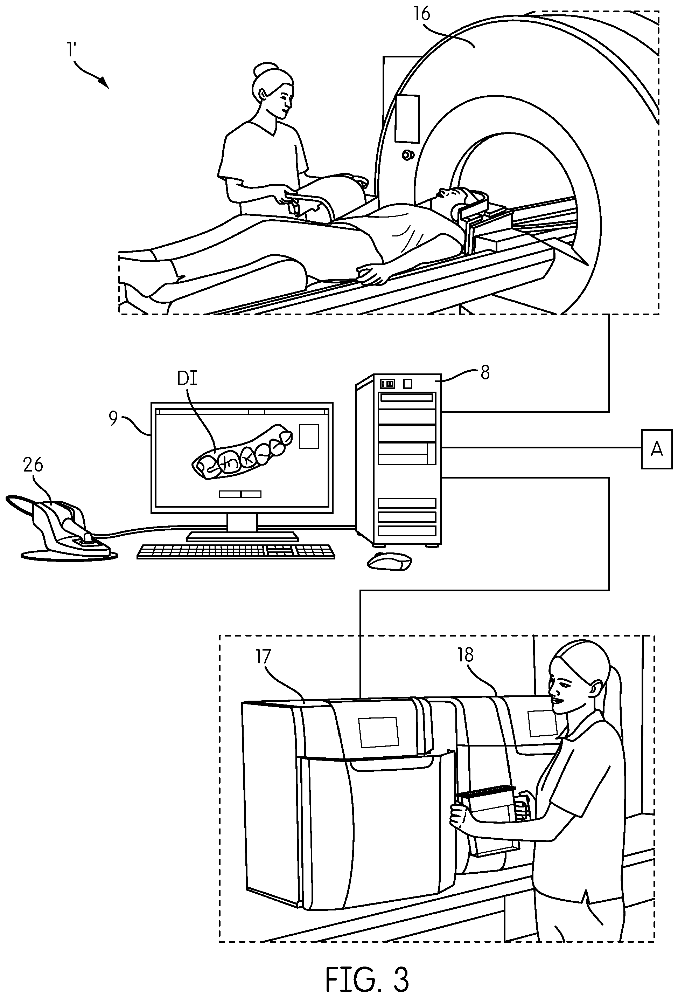

The computerized system (1') shown in Fig. 3 is a multifunctional system (1') for acquiring 2D images, 3D surface images, Volume images of the dental condition of the patient. The system (1') has a computer (8), a display (9), and input means such as keyboard and mouse. The computer (8) can be connected to various imaging devices such as a handheld optical intraoral scanner (26), a dental dedicated magneto resonance imaging device (16), and the X-ray imaging device (2) with DVT/PAN/CEPH modalities.

The system (1') is preferably configurable as an IoT (internet of things) system for cloud computing, data exchange, remote control, and the like, wherein the computer (8) can be bidirectionally connected via a local area network and/or the internet (not shown) to other dental devices such as the optical intraoral scanner (26), the MRI device (16), an x-ray imaging device (2), a dental milling machine (19), a 3D printing unit (17), a post processing units (18), and data storages (not shown). The systems (1,1') can be used by multiple users such as dentists.

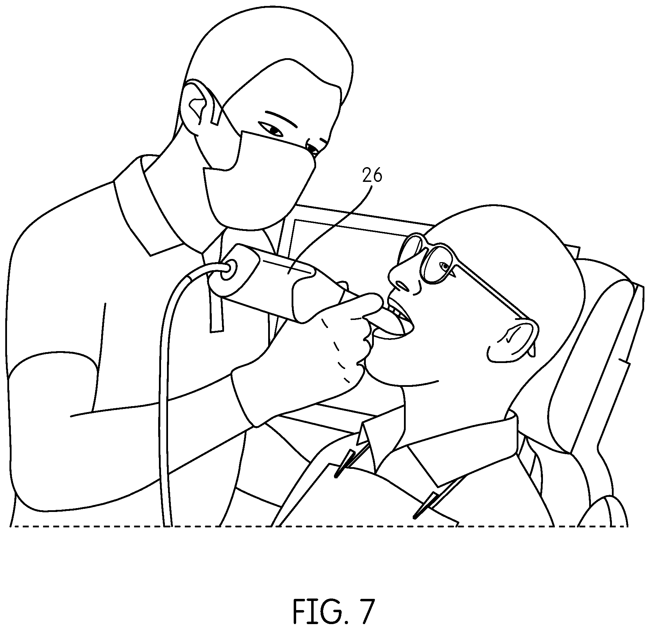

As shown in Fig. 7, the intraoral scanner (26) can be used for acquiring a digital impression (DI) of a patient' oral cavity and dental condition. Also, a wireless intraoral scanner can be used and connected to the system via wireless communication. The digital impression is a 3D surface image of the dental condition. The digital impression can serve to generate a digital 3D model for the design of restoration proposals. Other imaging modalities such MR imaging, X-ray imaging can also be used for generating a digital impression and/or a digital 3D model.

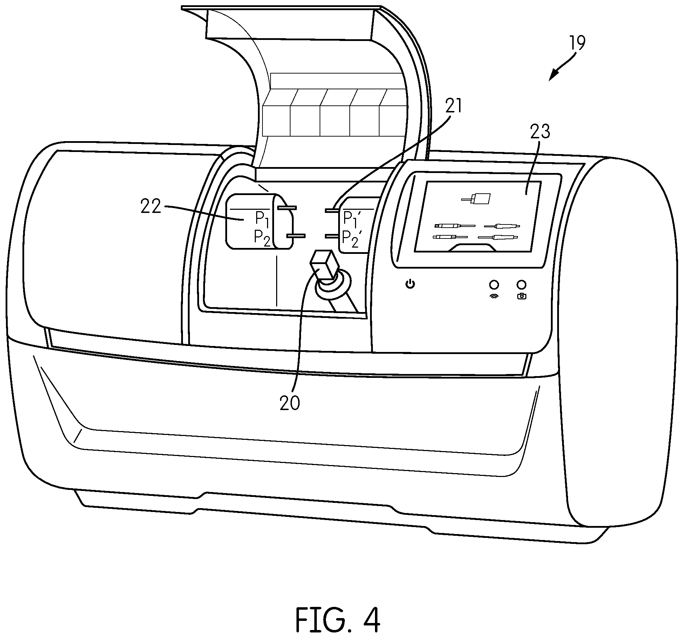

Fig. 4 shows the milling machine (19) for manufacturing a dental restoration design. The milling machine (19) comprises: a dental blank holder which holds a dental block (20) relatively movable with respect to the dental tools (21); two driving units / carriages (22) each movably holding one or more dental tools (21) for machining the dental blank (20); and a control unit adapted to control the dental blank holder and the driving units (22) based at least on a trajectory of the dental tools (21) relative to the dental blank (20) and a spatial amount of material removal from the dental blank (2) along the trajectory. As shown in Fig. 4, the milling machine (19) also has a user interface (touch screen). Various different dental tools (21) can be used to process the dental blank (20). The dental tools (21) may have micro-RFID tags at their rear side in a housing which can be recognized by the RFID reader/writer of the milling machine (19).

Fig. 3 shows a 3D printing unit (3D printer) according to one embodiment of the invention. The 3D printer according to the invention comprises a resin vat with an at least partially transparent bottom for receiving the liquid photoreactive resin for producing a solid component; a building platform for pulling out the component layer by layer out from the vat; a projector for projecting the layer geometry onto the transparent base; a transport apparatus for moving the building platform downward or upward in the vat. The 3D printer has a computer-implemented control unit (not shown) for controlling the overall operation. Fig. 3 also shows a post processing unit for post processing such as washing/drying/post exposure of the 3D objects printed by the (3D printer).

The software according to the invention allows high-quality automatic preparation of 3D printing or milling jobs for dental components such as splints, denture bases, models, restoration designs such as bridges and crowns and the like.

In the subsequent description, the computer-implemented method for generating an x-ray image of a patient's head will be explained in more detail. The extraoral x-ray system (1,1') has a control means configured to execute the method.

According to the method the x-ray device (2) has a first reference point (P1) for positioning a patient's oral cavity. The first reference point (P1) point can be set on the bite block (7) and will be explained later in more detail. The method comprises the following steps.

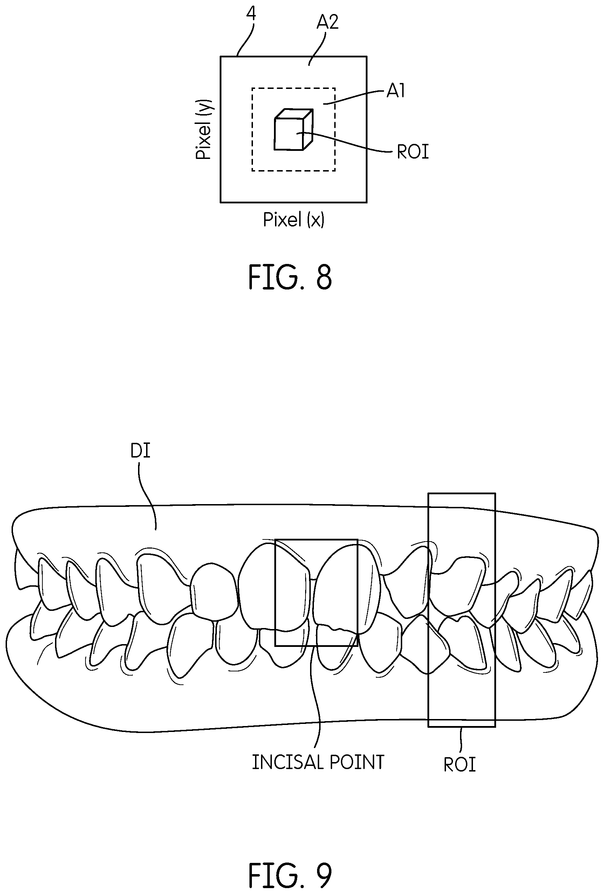

In an acquisition step a digital impression (DI) of the patient's oral cavity is acquired. This can be realized through the intraoral scanner (26). Alternatively, a digital impression can be retrieved from a data base. Fig. 9 shows an exemplary digital impression (DI) (3D data).

In a selection step, a region of interest (ROI) is selected in the digital impression (DI). The selection can be performed by the user manually via the software on the screen (9). Alternatively automatic selection can be performed via image processing for certain preset anatomical structures such as teeth.

In a determination step, the size of the region of interest as selected is determined by the software. This can be calculated from the 3D data of the digital impression.

In a further determination step a second reference point (P2) is determined in the digital impression. This is preferably an incisal point as shown in Fig. 9. Other points on the teeth / gingiva can be also used.

In a further determination step, the position of the selected region of interest is determined relative to the x-ray device (2) using the first reference point, the second reference point, and the digital impression. Since the patient bytes the dental block with the incisal point such calculation can be performed.

In a setting determination step, the positions of the x-ray detector and the x-ray source on opposing sides of the patient's head is set so that the region of interest (ROI) can be exposed with x-ray cone beam radiation and imaged with the x-ray detector.

In an adjusting step, the shutters (25) are adjusted so that a size of the aperture (24) restricts the x-ray cone beam to expose substantially not more than the region of interest (ROI) for the set positions of the x-ray detector and the x-ray source.

In an acquisition step at least one x-ray projection image of the region of interest (ROI) at the set positions of the x-ray detector and the x-ray source acquired with the correspondingly adjusted shutters (25).

In a reconstruction step, an x-ray image is reconstructed by using the at least one x-ray projection image.

Alternatively, the first reference point (P1) for positioning the patient's oral cavity can be set on the ceph unit via the head fixation (6') and/or a laser beam used to locate the patient's oral cavity. The ceph unit can also be provided with a bite block (not shown) to define a first reference point. The bite blocks' positions are known to the x-ray device (2).

In a preferred embodiment, the tube voltage of the x-ray source and/or the thickness of the shutters (25) are configured such that the shutters don't entirely absorb the x-ray radiation during the acquisition of the one or more x-ray projection images. Thereby, the x-ray projection images will include a first area (A1) showing the selected region of interest, and a second area (A2) showing an area surrounding the selected region of interest as shown in Fig.8. Thus, the second area will be darker and show the surrounding anatomical structure to the dentist.

In another preferred embodiment, alternatively the tube voltage of the x-ray source and/or the thickness of the shutters are configured such that the shutters entirely absorb the x-ray radiation during the acquisition of the one or more x-ray projection images. Thereby the x-ray projection images show only the selected region of interest.

In an embodiment, during the acquisition the x-ray detector (4) and the x-ray source (3) are moved along a curvilinear trajectory or a circular trajectory. And a plurality of x-ray projection images of the region of interest are acquired respectively at a plurality of set positions, along the said trajectory, of the x-ray detector and the x-ray source with the correspondingly adjusted shutters (25) as described above so that a size of the aperture (24) restricts the x-ray cone beam to expose substantially not more than the region of interest (ROI) for the set positions of the x-ray detector and the x-ray source.

In an alternative embodiment, during the acquisition the x-ray detector (14) and/or the x-ray source (3) are moved along a linear trajectory, wherein a plurality of x-ray projection images of the region of interest are acquired respectively at a plurality of set positions, along the said trajectory, of the x-ray detector (4) and the x-ray source (3) with the correspondingly adjusted shutters (25) as described above so that a size of the aperture (24) restricts the x-ray cone beam to expose substantially not more than the region of interest (ROI) for the set positions of the x-ray detector (14) and the x-ray source (3). The linear trajectory can be realized with the ceph unit. The linear trajectory can also be realized without the ceph unit by using actuators in the gantry to move the x-ray detector (4) and/or the x-ray source (3) linearly, however in a limited amount depending on the mechanism.

The generated x-ray image can be 3D volume image reconstructed form a plurality of x-ray projection images. The generated x-ray image can be a tomosynthesis image reconstructed form the plurality of x-ray projection images such as Panoramic image. The generated x-ray image can be cephalometric image.

## Claims

1. A method of generating an x-ray image using an x-ray system (1,1') comprising an x-ray device (2) which includes: an x-ray detector (4; 14); an x-ray source (3) for emitting a x-ray cone beam; an x-ray collimator (12; 15) having one or more adjustable shutters (25) for setting an aperture (24) for the x-ray radiation; and a first reference point (P1) for positioning a patient's oral cavity, the method comprising the step of:
acquiring a digital impression (DI) of the patient's oral cavity;
selecting a region of interest (ROI) in the digital impression (DI);
determining the size of the region of interest;
determining a second reference point (P2) in the digital impression;
determining the position of the selected region of interest relative to the x-ray device (2) using the first reference point, the second reference point, and the digital impression;
setting the positions of the x-ray detector and the x-ray source on opposing sides of the patient's head so that the region of interest can be exposed with x-ray cone beam radiation and imaged with the x-ray detector;
adjusting the shutters (25) so that a size of the aperture (24) restricts the x-ray cone beam to expose substantially not more than the region of interest (ROI) for the set positions of the x-ray detector and the x-ray source; and
acquiring at least one x-ray projection image of the region of interest (ROI) at each of the set positions of the x-ray detector and the x-ray source with the correspondingly adjusted shutters (25);
generating an x-ray image by using the one or more x-ray projection images.

2. The method according to claim 1, **characterized in that**
the tube voltage of the x-ray source and/or the thickness of the shutters (25) are configured such that the shutters don't entirely absorb the x-ray radiation during the acquisition of the one or more x-ray projection images, whereby the x-ray projection images includes a first area (A1) showing the selected region of interest, and a second area (A2) showing an area surrounding the selected region of interest.

3. The method according to claim 1, **characterized in that**
the tube voltage of the x-ray source and/or the thickness of the shutters are configured such that the shutters entirely absorb the x-ray radiation during the acquisition of the one or more x-ray projection images, whereby the x-ray projection images show only the selected region of interest.

4. The method according to any one of claims 1 to 3, **characterized in that**
during the acquisition the x-ray detector (4) and the x-ray source(3) are moved along a curvilinear trajectory or a circular trajectory, wherein a plurality of x-ray projection image of the region of interest are acquired respectively at a plurality of set positions, along the said trajectory, of the x-ray detector and the x-ray source with the correspondingly adjusted shutters (25).

5. The method according to any one of claims 1 to 3, **characterized in that**
during the acquisition the x-ray detector (4;14) and/or the x-ray source (3) are moved along a linear trajectory, wherein a plurality of x-ray projection images of the region of interest are acquired respectively at a plurality of set positions, along the said trajectory, of the x-ray detector and the x-ray source with the correspondingly adjusted shutters.

6. The method according to any one of claims 1 to 5, **characterized in that**
the generated x-ray image is a 3D volume image, a tomosynthesis image, or a cephalometric image.

7. A computer program comprising computer-readable codes which, when executed by a computerized x-ray system, causes the said system to perform the method steps of any of the preceding method claims.

8. An extraoral x-ray system (1), comprising:
an x-ray source (3); an x-ray detector (4;14); and a collimator (12;15);
a control means for controlling the x-ray source (3), the x-ray detector (4:14) and the x-ray collimator (12; 15), wherein the control means is configured to execute the method according to any one of the preceding claims.

## Amended claims

### Amended claims in accordance with Rule 137(2) EPC.

1. A method of generating an x-ray image using an x-ray system (1,1') comprising an x-ray device (2) which includes: an x-ray detector (4;14); an x-ray source (3) for emitting a x-ray cone beam; an x-ray collimator (12;15) having one or more adjustable shutters (25) for setting an aperture (24) for the x-ray radiation; and a first reference point (P1) for positioning a patient's oral cavity, the method comprising the step of:
acquiring a digital impression (DI) of the patient's oral cavity;
selecting a region of interest (ROI) in the digital impression (DI);
determining the size of the region of interest;
determining a second reference point (P2) in the digital impression, wherein the second reference point (P2) is an incisal point;
determining the position of the selected region of interest relative to the x-ray device (2) using the first reference point, the second reference point, and the digital impression;
setting the positions of the x-ray detector and the x-ray source on opposing sides of the patient's head so that the region of interest can be exposed with x-ray cone beam radiation and imaged with the x-ray detector;
adjusting the shutters (25) so that a size of the aperture (24) restricts the x-ray cone beam to expose substantially not more than the region of interest (ROI) for the set positions of the x-ray detector and the x-ray source; and
acquiring at least one x-ray projection image of the region of interest (ROI) at each of the set positions of the x-ray detector and the x-ray source with the correspondingly adjusted shutters (25);
generating an x-ray image by using the one or more x-ray projection images.

2. The method according to claim 1, **characterized in that**
the tube voltage of the x-ray source and/or the thickness of the shutters (25) are configured such that the shutters don't entirely absorb the x-ray radiation during the acquisition of the one or more x-ray projection images, whereby the x-ray projection images includes a first area (A1) showing the selected region of interest, and a second area (A2) showing an area surrounding the selected region of interest.

3. The method according to claim 1, **characterized in that**
the tube voltage of the x-ray source and/or the thickness of the shutters are configured such that the shutters entirely absorb the x-ray radiation during the acquisition of the one or more x-ray projection images, whereby the x-ray projection images show only the selected region of interest.

4. The method according to any one of claims 1 to 3, **characterized in that**
during the acquisition the x-ray detector (4) and the x-ray source(3) are moved along a curvilinear trajectory or a circular trajectory, wherein a plurality of x-ray projection image of the region of interest are acquired respectively at a plurality of set positions, along the said trajectory, of the x-ray detector and the x-ray source with the correspondingly adjusted shutters (25).

5. The method according to any one of claims 1 to 3, **characterized in that**
during the acquisition the x-ray detector (4;14) and/or the x-ray source (3) are moved along a linear trajectory, wherein a plurality of x-ray projection images of the region of interest are acquired respectively at a plurality of set positions, along the said trajectory, of the x-ray detector and the x-ray source with the correspondingly adjusted shutters.

6. The method according to any one of claims 1 to 5, **characterized in that**
the generated x-ray image is a 3D volume image, a tomosynthesis image, or a cephalometric image.

7. A computer program comprising computer-readable codes which, when executed by a computerized x-ray system, causes the said system to perform the method steps of any of the preceding method claims.

8. An extraoral x-ray system (1), comprising:
an x-ray source (3); an x-ray detector (4;14); and a collimator (12;15);
a control means for controlling the x-ray source (3), the x-ray detector (4:14) and the x-ray collimator (12;15), wherein the control means is configured to execute the method according to any one of the preceding claims.
